# EUROPEAN PATENT APPLICATION

(11) **EP 4 014 992 A1**
(43) Date of publication of application: **22.06.2022**
(21) Application number: 20216201.2
(22) Date of filing: 21.12.2020
(51) Int. Cl.: A61K 39/275, C12N 15/86

(54) **MODIFIED PARAPOXVIRUS HAVING INCREASED IMMUNOGENICITY**

(71) Applicant: Eberhard Karls Universität Tübingen Medizinische Fakultät, 72074 Tübingen (DE)
(72) Inventor: Amann, Ralf, 72070 Tübingen (DE); Salomon, Ferdinand, 72072 Tübingen (DE)
(74) Representative: Witte, Weller & Partner Patentanwälte mbB

(57) **Abstract**

The present invention relates to a modified *Parapoxvirus,* preferably a *Parapoxvirus* vector, having an increased immunogenicity, a biological cell containing said modified *Parapoxvirus,* a pharmaceutical composition, preferably a vaccine, containing said modified *Parapoxvirus* vector and/or said cell, and a new use of said modified *Parapoxvirus.*

SEQUENCE LISTING

<110>  Eberhard Karls Universität, Medizinische Fakultät

<120>  Parapoxvirus vector having increased immunogenicity

<130>  5402P617EP

<160>  8

<170>  PatentIn version 3.5

<210>  1
<211>  16
<212>  DNA
<213>  Artificial Sequence

<220>
<223>  eP1 Promoter

<400>  1
aaaaattgaa aaatta                                                    16


<210>  2
<211>  16
<212>  DNA
<213>  Artificial Sequence

<220>
<223>  eP2 Promoter

<400>  2
aaaaattgaa attcta                                                    16


<210>  3
<211>  16
<212>  DNA
<213>  Artificial Sequence

<220>
<223>  "Optimized Early" Promoter

<400>  3
aaaaattgaa aaayta                                                    16


<210>  4
<211>  15
<212>  DNA
<213>  Artificial Sequence

<220>
<223>  7.5 kDa Promoter

<400>  4
aaaagtagaa aatta                                                     15


<210>  5
<211>  16
<212>  DNA

<213>  Artificial Sequence

<220>
<223>  VEGF Promoter

<400>  5
caaaatgtaa attata                                                    16


<210>  6
<211>  2761
<212>  DNA
<213>  Artificial Sequence

<220>
<223>  D1701-V ORF125 - 126 - 127

<400>  6
atggcaaaca gagaagaaat tgacgcctct gccgtcatgg ctgcctacct cgcgagagag      60

tacgcggtgg ctgtagagga acagctgacg ccgcgcgagc gcgatgcgct cgaagccctt     120

cgcgtttccg gcgaggaggt ccggtcgccg ctgctgcaag aactctcgaa cgcgggcgag     180

caccgcgcca accccgaaaa ctcgcacatc cccgccggcc tcgtctccgc gcttctcgaa     240

gcccccacct cccccggccg catggtcact gcgattgagc tctgcgcgca gatgggccgg     300

ctatggacgc gcggccacaa gctcgttgac ttcatgcggc tcgtgtacgt gctcctagac     360

cgtctgccgc ccacggccga cgaggacctc agcgcctggc tgcaggccgt cgcgcgagtg     420

cacggcacgc ggcgccgcct gcaccgcgtt ctcggcgtcg gggccgtcgt ggcaggcgtc      480

ggtatgctgc tgctcggcgt gcgcgtgttg cggcgcacat aactttttat ctcggctcaa     540

actgaaatac gacattggac tacgaaatct ataattttgc gcacggcctt cggcgtaaga     600

taggataaat aacctctaag caacaagcat ggccgatgag agagaggccg acggcgcgct     660

gttccggtac ctggagagcg aggaccgtcc ggacgtggag cacatgcgcc ggctgctgga     720

cgagggcgcg gacgtgaact acgcgggccc gcgcgggtac gcgccgctgc acatgctcat     780

gcgcagcaac ccgctagacc ccgacgcggt gcgactgctg ctcgccgcgg gcgcagacgt     840

gaacgcaaca tcgctctgcg ggttcacgcc gctgcactca tacatgtgct tcgggaccgt     900

gacgccagac acgctgcgtg cgctcatgcg ccacggcgca agcgtcagcg acctcgagcg     960

caacatcaac gcgctgatcg agtacttcaa ccgcgacggc tgcatgggcg gcgcggaggc    1020

ggccgtgatc gcactgctgg tggagcacgg cgcgtacgtg aacgccaaag acgaccttgg    1080

acgaacgccg ctgcacatct acctgtccgg cttcttcgtg tcggcaccgg tggcgctagc    1140

gctgatcgcg ctcggcgcga acccgaacgc cacggacgcg tacgggcgca cgccactgca     1200

cgccttcctg cgctcccgcg acgtggaccc cgctgtgctg aagacgctca tcgccgcggg    1260

cgcagacccg ctcgcgcgcg acatcatccg gcgcacggcg ctgcactacc actgcgagtc    1320

cttcaagacg cgcgctagcg tcatcgagac gctggtggcc gccggctgcg accccgcgag    1380

cacagacctg ctcgacaaca cagcgctaca cagcatggcc atgggcagct cctgtcgcgc 1440

ttcgctgatc cgcccgctgc tggccgcggg cgtgtccgtg aacgcgcgca acgcgcggct 1500

gcagacgccg ctgcaccttg cggccgtgtt taacccgccg gcctgcgcgc ggctgctggc 1560

cgcgggcgcg gaccccgcgc tcgcagacct ggacgagaca acgccgctgc tgagcatggt 1620

gcgacacaac tgcgcgcgcg cgctgcgcac agcgctgcca ttggcgccgg acgcgctagt 1680

ggccggcgcg gtgaaccgcg tgaacgcgcg cacgccgagc gcggccacgc gcgagtgcgt 1740

gatggcgctg gcgctgcgcg gcgcgctgga cctgctgagc gcggagagcg cgcgagcgca 1800

cgcggctgcg atccgcgcct gtgaggcgga ggtcgcgctg ctgcggcgca cgcgcctggg 1860

cgcgccgccg acgacgctct tcgcgctgct ggcaggacga ccgaacacgc tggtttccgc 1920

aaaggcggcg cgccgagcga tggcagacgt gtgtgtctac cgtgcggcgc tggccgcgag 1980

cgtggagcgc gtgcgccgaa agtcctcgct ggtcgagcgc ctcaccgcga tggtgtgtcc 2040

gtgcgctctg ccgccagagc tagtgacgcg catcctcgcg ctcctgaccg tggaggaact 2100

ctcttgcgca atgcgcaaat aataatgaac tataactagg cttattagag gcactatttg 2160

tgcagagtcg ttagttatag ttatagttat acttacaatt ggaatgtcga acaacaaaat 2220

tctggtgtgt gttgcgatta ttcttactta tacattatac acaaatgcgt attgtgttga 2280

gtatttggaa agtgaggagg ataaacaaca gtgcggtagt aatggtgcgt cttcgagttc 2340

accacacatg ctcagagaac tcagggccgc gttcggaaag gtaaaaactt tcttccagat 2400

gaaagaccaa ctgaacagta tgctactcac acagtcgctc ctcgacgact tcaaaggcta 2460

cctcgggtgt caggcacttt ccgagatgat acagttttac ttggaagagg tgatgccgca 2520

ggcggaaaat cacgggccgg acgtcaaaga gcacgttaac tcgctgggag aaaagctcaa 2580

aacgctgcgt cttcgactgc gtcgctgcca ccgcttcctg ccgtgtgaga acaagagtaa 2640

ggccgtggcg caagtcaaac gcgtgttcaa catgctgcag gaacgaggtg tttacaaggc 2700

catgagcgag ttcgacatat tcatcaacta catagaatca tacatgacta ctaaatcata 2760

a 2761

<210> 7
<211> 2057
<212> DNA
<213> Artificial Sequence

<220>
<223> pDel 126-4-2-AcGFP

<400> 7
gaggaacagc tgacgccgcg cgagcgcgat gcgctcgaag cccttcgcgt ttccggcgag 60

gaggtccggt cgccgctgct gcaagaactc tcgaacgcgg gcgagcaccg cgccaacccc 120

gaaaactcgc acatccccgc cggcctcgtc tccgcgcttc tcgaagcccc cacctcccct 180

ggccgcatgg tcactgcgat tgagctctgc gcgcagatgg gccggctatg gacgcgcggc 240

cacaagctcg ttgacttcat gcggctcgtg tacgtgctcc tagaccgtct gccgcccact 300

gccgacgagg acctcagcgc ctggctgcag gccgtcgcgc gagtgcacgg cacgcggcgc 360

cgcctgcacc gcgttctcgg cgtcggggcc gtcgtggcag gcgtcggtat gctgctgctc 420

ggcgtgcgcg tgttgcggcg cacataagat atcaaaaatg taaattctaa tacattaaat 480

tataggatcc ggtaccacta gtcttaagat cgatttttta tcttgttttt atcctgtctt 540

tttatcagtt ttttagctag ttaaaacata aatagtaaag ctaaaaagag actatatcgg 600

cggctggagt cttgcaacaa ccagctccgg aaaaaattga aattctaact tgtgttctta 660

taaatgatca attgagatct ttcgaaaccg gtcgccacca tggtgagcaa gggcgccgag 720

ctgttcaccg gcatcgtgcc catcctgatc gagctgaatg gcgatgtgaa tggccacaag 780

ttcagcgtga gcggcgaggg cgagggcgat gccacctacg gcaagctgac cctgaagttc 840

atctgcacca ccggcaagct gcctgtgccc tggcccaccc tggtgaccac cctgagctac 900

ggcgtgcagt gcttctcacg ctaccccgat cacatgaagc agcacgactt cttcaagagc 960

gccatgcctg agggctacat ccaggagcgc accatcttct tcgaggatga cggcaactac 1020

aagtcgcgcg ccgaggtgaa gttcgagggc gataccctgg tgaatcgcat cgagctgacc 1080

ggcaccgatt tcaaggagga tggcaacatc ctgggcaata agatggagta caactacaac 1140

gcccacaatg tgtacatcat gaccgacaag gccaagaatg gcatcaaggt gaacttcaag 1200

atccgccaca acatcgagga tggcagcgtg cagctggccg accactacca gcagaatacc 1260

cccatcggcg atggccctgt gctgctgccc gataaccact acctgtccac ccagagcgcc 1320

ctgtccaagg accccaacga gaagcgcgat cacatgatct acttcggctt cgtgaccgcc 1380

gccgccatca cccacggcat ggatgagctg tacaagtgag cggccgctga tcaacgcgtt 1440

ttttatcctg cagggctagc cggccggcat gctttttatg cgatcgcatg tcgaacaaca 1500

aaattctggt gtgtgttgcg attattctta cttatacatt atacacaaat gcgtattgtg 1560

ttgagtattt ggaaagtgag gaggataaac aacagtgcgg tagtaatggt gcgtcttcga 1620

gttcaccaca catgctcaga gaactcaggg ccgcgttcgg aaaggtaaaa actttcttcc 1680

agatgaaaga ccaactgaac agtatgctac tcacacagtc gctcctcgac gacttcaaag 1740

gctacctcgg gtgtcaggca ctttccgaga tgatacagtt ttacttggaa gaggtgatgc 1800

cgcaggcgga aaatcacggg ccggacgtca aagagcacgt taactcgctg ggagaaaagc 1860

tcaaaacgct gcgtcttcga ctgcgtcgct gccaccgctt cctgccgtgt gagaacaaga 1920

gtaaggccgt ggcgcaagtc aaacgggtgt tcaacatgct gcaggaacga ggtgtttaca 1980

aggccatgag cgagttcgac atattcatca actacataga atcatacatg actactaaat 2040

cataaaaatg tatataa                                                2057

<210>  8
<211>  1132
<212>  DNA
<213>  Artificial Sequence

<220>
<223>  pDel-126

<400>  8
gaggaacagc tgacgccgcg cgagcgcgat gcgctcgaag cccttcgcgt ttccggcgag      60

gaggtccggt cgccgctgct gcaagaactc tcgaacgcgg gcgagcaccg cgccaacccc     120

gaaaactcgc acatccccgc cggcctcgtc tccgcgcttc tcgaagcccc cacctcccct     180

ggccgcatgg tcactgcgat tgagctctgc gcgcagatgg gccggctatg gacgcgcggc     240

cacaagctcg ttgacttcat gcggctcgtg tacgtgctcc tagaccgtct gccgcccact     300

gccgacgagg acctcagcgc ctggctgcag gccgtcgcgc gagtgcacgg cacgcggcgc     360

cgcctgcacc gcgttctcgg cgtcggggcc gtcgtggcag gcgtcggtat gctgctgctc     420

ggcgtgcgcg tgttgcggcg cacataagat atcaaaaatg taaattctaa tacattaaat     480

tataggatcc ggtaccacta gtatcgatac gcgtttttta tcctgcaggg ctagccggcc     540

ggcatgcttt ttatgcgatc gcatgtcgaa caacaaaatt ctggtgtgtg ttgcgattat     600

tcttacttat acattataca caaatgcgta ttgtgttgag tatttggaaa gtgaggagga     660

taaacaacag tgcggtagta atggtgcgtc ttcgagttca ccacacatgc tcagagaact     720

cagggccgcg ttcggaaagg taaaaacttt cttccagatg aaagaccaac tgaacagtat     780

gctactcaca cagtcgctcc tcgacgactt caaaggctac ctcgggtgtc aggcactttc     840

cgagatgata cagttttact tggaagaggt gatgccgcag gcggaaaatc acgggccgga     900

cgtcaaagag cacgttaact cgctgggaga aaagctcaaa acgctgcgtc ttcgactgcg     960

tcgctgccac cgcttcctgc cgtgtgagaa caagagtaag gccgtggcgc aagtcaaacg    1020

ggtgttcaac atgctgcagg aacgaggtgt ttacaaggcc atgagcgagt tcgacatatt    1080

catcaactac atagaatcat acatgactac taaatcataa aaatgtatat aa            1132

## Description

The present invention relates to a modified *Parapoxvirus,* preferably a *Parapoxvirus* vector, having an increased immunogenicity, a biological cell containing said modified *Parapoxvirus,* a pharmaceutical composition, preferably a vaccine, containing said modified *Parapoxvirus* vector and/or said cell, and a new use of said modified *Parapoxvirus.*

Modified viruses, e.g. viral vectors, have multiple applications in biosciences, medicine and process engineering. For instance, viral vector-based vaccines are promising to elicit cellular and humoral immune responses to any kind of antigen peptides. Within the genus *Parapoxvirus* of the *Poxviridae* the *Parapoxvirus ovis* (Orf virus; ORFV) strain D1701-V comprises various properties particularly favorable for the development of a vector platform technology and was shown to facilitate various vaccination approaches.

One problem which can be found in the art of modified viruses or viral vectors, however, is low immunogenicity. The low immunogenicity reduces the effectivity of viral vector-based vaccines. Current strategies to address this problem use the implementation of immuno-modulatory elements or the formulation of the viral vectors in conjunction with pharmacological or immunological agents which improve the immune response of a vaccine. However, these approaches have not been fully successful. Some viral vectors are of limited packaging capacity and the expression of immunomodulatory elements quite often render the viruses inoperable. Furthermore, adjuvants are frequently toxic to the vaccinated organism that is why viral vector-based vaccination is often associated with side effects.

Against this background there is a need in the art to provide new modified viruses, in particular viral vectors, which can be used for the effective production of viral vector-based vaccines and other applications, where the problems known in the art are reduced or even avoided.

Therefore, it is an object underlying the invention to provide such a modified virus or viral vector, respectively, which is characterized by an increased immunogenicity without requiring the inclusion of immunomodulatory elements or the formulation of the vector with adjuvants.

The invention satisfies these and other needs by providing a modified *Parapoxvirus,* preferably a *Parapoxvirus* vector, comprising at least one functional mutation in the viral open reading frame (ORF) encoding the 'Ankyrin Repeat 1' (ANK-1), wherein said vector comprises increased immunogenicity in comparison with the same vector without said functional mutation.

According to the invention, *'Parapoxvirus'* is a genus of viruses in the family of *Poxviridae* and the subfamily of *Chordopoxvirinae.* Like all members of the family *Poxviridae* they are oval, relatively large, double-stranded DNA viruses. Parapoxviruses have a unique spiral coat that distinguishes them from other poxviruses. Parapoxviruses infect vertebrates, including a wide selection of mammals, and humans. According to the invention all kinds of Parapoxviruses are suitable, however, Orf viruses are of preference.

According to the invention, 'modified' *Parapoxvirus* refers to a *Parapox*virus-derived virus which has been technically modified over the wild type counterpart.

According to the invention a *'Parapoxvirus* vector' refers to a vector or plasmid based on or consisting of the *Parapoxvirus* genome, which is configured for the transfection of biological cells, preferably of mammalian and human cells, and further preferably also for the transport and/or the expression of a transgene or foreign gene in(to) biological cells.

According to the invention, 'Ankyrin Repeat 1' (ANK-1) belongs to a group of protein motives consisting of two alpha helices separated by loops, first discovered in signaling proteins in yeast Cdc10 and Drosophila Notch. However, Ankyrin Repeats (ANK) are ubiquitous throughout the kingdoms of life, however, absent in most viruses except the poxvirus family, specifically the Chordopoxviruses. The motif consists of tandem repeated consensus domains that are crucial to facilitate protein-protein interactions and is found in four proteins encoded by ORFV D1701-V (ORF123, -126 (ANK-1), -128 (ANK-2) and -129 (ANK-3)). While plenty of cellular ANK proteins are described to be involved in cell-cell signaling, cytoskeleton integrity, regulation of transcription and cell cycle, inflammatory response, or protein transport, only little is known about the function of poxviral ANK proteins. It has been demonstrated that these four proteins harbor F-box-like motifs that may recruit specific substrates to SCF1 ubiquitin ligases via a substrate-binding domain and thereby exploit the cell's ubiquitin-proteasome machinery. One of these proteins, the ORF126 encoded ANK-1 protein, was shown to co-localize with the mitochondria via the essential ankyrin repeats 8 and 9. Nevertheless, the physiological consequences have not been demonstrated yet. Furthermore, a recent study reported an influence of ORFV encoded ANK proteins on the Hypoxia-inducible factor (HIF) pathway. While this pathway plays a crucial role in the regulation of cellular responses to hypoxia, downstream targets like angiogenesis- and anti-apoptotic programs favor viral pathogenesis. It has been shown that the factor inhibiting HIF (FIH) was sequestered by ANK proteins upon ORFV infection resulting in enhanced HIF activity.

According to the invention, a 'functional mutation' refers to a genetic alteration of a gene and the encoded protein which, in comparison to the non-mutated wild type, results in a change of activity and/or function. The functional mutation includes, without being limited thereto, knockout, point, deletion, frame shift, and substitution mutations etc., which all result in the alteration of ANK-1 function, whether a dysfunctional ANK-1 will be the outcome or the expression of ANK-1 is suppressed, reduced or avoided, respectively.

According to the invention, 'immunogenicity' is the ability of the *Parapoxvirus* vector to provoke an immune response in the body of an animal of human being, i.e. the ability to induce humoral and/or cell-mediated immune responses. The immunogenicity can be measured by various methods well known to the skilled person. An overview on such methods can be found in Madhwa et al. (2015), Immunogenicity assessment of biotherapeutic products: An overview of assays and their utility, Biologicals Vol. 43, Is. 5, pp. 298-306.

The inventors were able to realize that a *Parapoxvirus* vector having a functionally mutated, preferably inactivated ANK-1, is characterized by a particular solid and elevated immunogenicity in comparison with a non-mutated counterpart vector or wild type virus, respectively. Increased immunogenicity was demonstrated by the vector's ability to activate peripheral blood mononuclear cells, or to induce antigen-specific immune responses in vitro. At the same time, the inventors found that the modified *Parapoxvirus* or *Parapoxvirus* vector according to the invention still exhibits excellent growth behavior and has the capability of expressing trans- or foreign genes. The analyses performed by the inventors demonstrate a high potential for the design of vector-based vaccines.

Due to the identified increased immunogenicity the modified *Parapoxvirus* or *Parapoxvirus* vector according to the invention are also well-qualified for other applications, such as oncolytic virus or vector, immune stimulant, a tool for gene therapy or an inactivated virus, respectively.

The findings of the inventors were surprising and could not be expected by the skilled person. It could be assumed that the ORF encoding the ANK-1 is essential or at least favorable for the functioning and replicability or the *Parapoxvirus* genome and, consequently, for a *Parapoxvirus-based* vector. In other words, it could have been assumed that in a *Parapoxvirus* vector it cannot be dispensed of the ORF encoding the ANK-1. Furthermore, a functional mutation in a viral ORF typically results in a loss of immunogenicity and it could have been expected the same effect when mutating the ORF encoding ANK-1 in *Parapoxviruses.* The art, therefore, points into a direction opposite to the one the invention points to.

The problem underlying the invention is fully achieved herewith.

In an embodiment of the invention the functional mutation results in a reduction of the activity of ANK-1 over non-mutated ANK-1.

This embodiment includes any kind of genetic alteration in the ORF encoding ANK-1 which results in a loss of function of ANK-1 (loss-of-function mutation) or, in comparison with the wild type counterpart, a significant reduction of ANK-1 expression or activity, respectively. Therefore, this embodiment encompasses a downregulation of ANK-1 function which need not be a complete inactivation. However, a complete inactivation or cativity reduction to zero of ANK-1 is preferred in an embodiment of the invention. The mutation includes, without being limited thereto, knockout, point, deletion, frame shift, and substitution mutations etc., which all result in the inactivation of ANK-1 function, whether a dysfunctional ANK-1 will be the outcome or the expression of ANK-1 is suppressed, reduced or avoided, respectively.

In an embodiment of the invention the modified *Parapoxvirus* is a *Parapoxvirus ovis* (*Orf* virus, ORFV) or the *Parapoxvirus* vector is a *Parapoxvirus ovis* (*Orf* virus, ORFV) vector.

The *Parapoxvirus ovis* or *Orf* virus (ORFV) is the prototype of the genus of the *parapoxviruses* and belongs to the family of the *Poxviridae.* ORFV are enveloped, complex dsDNA viruses having a morphology that reminds of a ball of wool and have an average size of approx. 260 x 160 nm. They comprise a linear DNA genome with high GC content and a size of approx. 130 to 150 kbp, the central region of which is delimited on both sides by ITR regions ("inverted terminal repeats") and ends in a hair-pin structure, which covalently links both DNA single strands to each other. In the central region of the genome there are predominantly genes which are mainly essential for the viral replication and morphogenesis and which are highly conserved among the *poxviruses.* In contrast, in the ITR regions there are so-called non-conserved virulence genes which significantly determine the host range, the pathogenicity and the immunomodulation and, therefore, characterize the virus.

ORFV has a variety of characteristics which makes it interesting for the production of recombinant vaccines and prefers it over other technologies. In comparison to *orthopoxviruses* ORFV is characterized by a very narrow natural host tropism which includes sheep and goats. As a consequence, an inhibiting "preimmunity" against the vector in humans, which is caused by a natural infection, as it can be observed in the most common viral vectors of the *vaccinia* and *adenoviruses,* can be almost excluded. Furthermore, the exceptionally weak and short-lived ORFV specific vector immunity allows a very effective booster and/or refreshing vaccination or immunization with ORFV based vaccines which are directed against further pathogens.

In another embodiment of the invention said ORFV is of the strain D1701, preferably of the strain D1701-V.

This measure has the advantage that such a virus or vector is used which is attenuated and causes only asymptotic infections in the host. According to the invention all variants of D1701 are covered, including D1701-B and D1701-V while the latter is preferred. The characteristics of the strain D1701-V are disclosed in Rziha et al. (2019; *I.c.*).

In another embodiment, said ANK-1 is encoded by viral open reading frame 126 (ORF126), preferably said open reading frame (ORF) is located at the nucleotide positions nt 22.055 ± 100 to nt 23.546 ± 100.

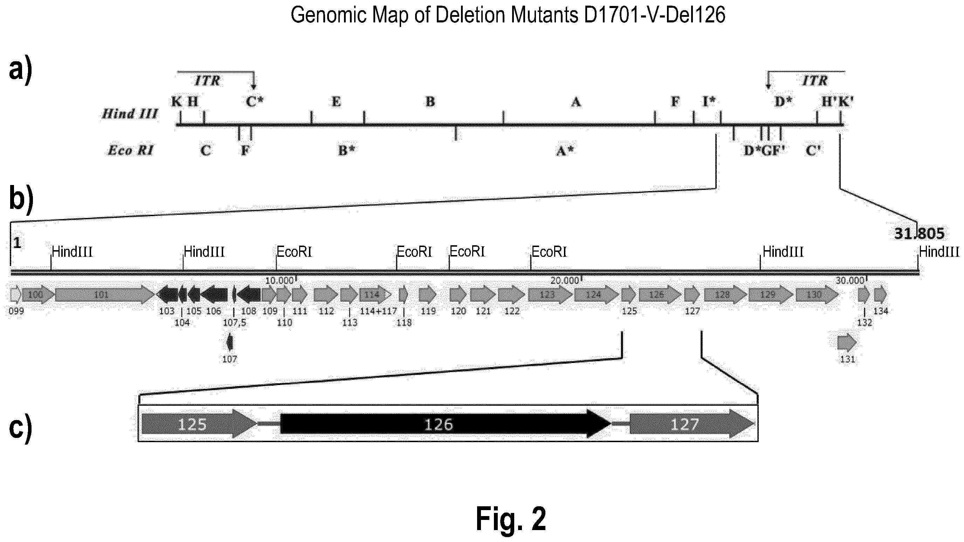

With this measure, the particular open reading frame is provided as the target for inactivation, which in ORFV D1701 encodes the ANK-1. The skilled person, therefore, receives detailed information of the place of mutation. In this context the indicated nucleotide positions refer to a 31.805 nt comprising DNA sequence encoded by the right hand part of the ORFV D1701-V genome as determined by Rziha, H.-J., unpublished data as indicated in Figure 2b.

In a further embodiment the modified *Parapoxvirus* or *Parapoxvirus* vector according to the invention further comprises:
(1) at least one nucleotide sequence encoding a transgene, and
(2) at least one promoter controlling the expression of the transgene-encoding nucleotide sequence.

According to the invention a 'transgene', or synonymously a foreign gene, refers to a gene or open reading frame (ORF) which does not originate from the Parapoxvirus genome.

According to the invention a 'promoter' refers to such a nucleic acid section which allows the regulated expression of the transgene in the Parapoxvirus vector of the invention. Preferably it refers to an ORFV promoter, i.e. a promoter existing in the wild type ORFV genome or a promoter derived therefrom, or an artificial promoter, such as a poxvirus promoter, CMV promoter etc.

This measure has the advantage that the virus or vector according to the invention is used as a genetic tool for the production of any foreign protein, such as an antigen. Together with the vector's excellent immunogenicity properties such embodiment improves the suitability of the virus or vector according to the invention as active substance or component of a vaccine composition.

In a yet further embodiment of the invention said transgene-encoding nucleotide sequence is inserted into said ANK-1-encoding viral ORF, and/or, alternatively, said ANK-1-encoding viral ORF is replaced by said transgene encoding nucleotide sequence.

According to the invention 'inserted into said ANK-1-encoding viral ORF' means that the ANK-1-encoding sequence or open reading frame, respectively, is deleted or opened-up at any position and the transgene-encoding sequence along with its promoter is inserted. Sections of the ANK-1-encoding sequence may or may not be removed by said procedure. According to the invention 'replaced by said transgene encoding nucleotide sequence' means that the entire ANK-1-encoding viral ORF is removed from the virus or vector and the sequence gap receives the transgene-encoding nucleotide sequence along with its promoter. This embodiment has the advantage that the insertion of the transgene-encoding nucleotide sequence provides for the functional mutation or inactivation of ANK-1.

In another embodiment said modified Parapoxvirus or Parapoxvirus vector according to the invention comprises more than one transgene-encoding nucleotide sequence, preferably the number of transgene-encoding nucleotide sequences is selected from the group consisting of: 2, 3, 4 or more.

In this embodiment the respective transgenes or transgene-encoding sequences can all be located in the ANK-1-encoding viral ORF. However, alternatively, the various transgene-encoding sequences can also be located elsewhere at the same or different locations in the virus or vector genome or construct, respectively. In every insertion locus multiple foreign genes, preferably 2, 3, 4 or more, can be expressed. For example, in ORFV D1701 or D1701-V the transgene-encoding sequences can preferably be located in any of the viral ORFs 112, 119, 126, etc.

This measure has the advantage that by means of a single modified Parapoxvirus or Parapoxvirus vector according to the invention multiple foreign or transgenes can be expressed. This embodiment is especially appropriate for the production of polyvalent vaccines which, at the same time, are directed against a number of antigenic structures.

In another embodiment of the modified Parapoxvirus or Parapoxvirus vector according to the invention the promoter is an early ORF promoter, which preferably comprises a nucleotide sequence which is selected from: SEQ ID NO: 1 (eP1), SEQ ID NO: 2 (eP2), SEQ ID NO: 3 (optimized "early"), SEQ ID NO: 4 (7.5 kDa promoter), and SEQ ID NO: 5 (VEGF).

This measure has the advantage that such promoters are used which allow a high expression level of the transgene and a targeted control of the expression. The promoters eP1 and eP2 were developed by the inventors and are published in Rziha, H.-J., et al. (2019; I.c.). The remaining promoters originate from the vaccinia virus and are described in other connections in Davidson and Moss (1989), Structure of Vacciniavirus late promoters, J. Mol. Biol., Vol. 210, pp- 771-784, and Yang et al. (2011), Genome-wide analysis of the 5' and 3' ends of Vaccinia Virus early mRNAs delineates regulatory sequences of annotated and anomalous transcripts, J. Virology, Vol. 85, No. 12, pp. 5897-5909, Broyles (2003), Vaccinia virus transcription, J. Gene. Virol., Vol. 84, No. 9, pp. 2293-2303. According to the findings of the inventors P2 causes a significantly increased expression strength in comparison to eP1. This was surprising because the promoter eP1 corresponds by 100% to the consensus sequence from the Vacciniavirus, however not eP2. The low expression of the "optimum" Vacciniavirus promoter (Orthopox) in ORFV (Parapox) is a contradiction and surprising. It is also surprising that the eP2 promoter results in a strong expression.

In another embodiment of the modified *Parapoxvirus* or *Parapoxvirus* vector according to the invention the transgene is selected from the group of the following antigens:
- viral antigen, preferably
   severe acute respiratory syndrome coronavirus 2 (SARS-CoV-2) antigen, including spike (S), envelope (E), and nucleocapsid (N) proteins;
   rabies virus antigen, including glycoprotein (RabG);
   influenza A antigen, including nucleoprotein (NP), hemagglutinin (HA), neuraminidase (NA);
- tumor antigen, preferably viral tumor antigen, including HPV selective viral tumor antigen;
- tumor associated antigen, including viral tumor associated antigen, including HPV selective viral tumor-associated antigen;
- parasitic antigen, preferably plasmodium antigen;
- cytokine;
- protein originated or derived from a mammal, preferably from a mammal recipient.

This measure has the advantage that especially important antigens, in particular for the production of vaccines, are expressible via the modified Parapoxvirus or Parapoxvirus vector according to the invention.

Another subject matter of the present invention relates to a biological cell, preferably a mammalian cell, further preferably a Vero cell, a HEK 293 cell or an antigen-presenting cell, containing the modified Parapoxvirus or Parapoxvirus vector according to the invention.

Vero and HEK 293 cells are currently used for the production of the modified virus or vector according to the invention. In the host, however, the virus is been taken up be antigen-presenting cells.

Another subject matter of the present invention relates to a composition, preferably a pharmaceutical composition, containing the modified Parapoxvirus or Parapoxvirus vector of the invention and/or the cell of the invention, and a pharmaceutically acceptable carrier. The pharmaceutical composition can be preferably a vaccine, further preferably a polyvalent vaccine, an immune stimulant, a tool for gene therapy, etc.

Pharmaceutically acceptable carriers are well known in the art. They include, without being limited thereto, stabilizers, binders, diluents, salts, adjuvants, buffers, lipids, etc. An overview can be found in Rowe (2020), Handbook of Pharmaceutical Excipients, 9th edition, Pharmaceutical Press.

The characteristics, advantages, features, and further developments of the modified Parapoxvirus or the Parapoxvirus vector according to the invention apply to the cell of the invention and the composition of the invention in a corresponding manner.

Another subject-matter of the invention relates to the use of a modified Parapoxvirus or a Parapoxvirus vector comprising at least one inactivating mutation in the viral open reading frame (ORF) encoding ANK-1 for the induction of an immune response in a living being, preferably a mammal or a human being.

The characteristics, advantages, features, and further developments of the modified Parapoxvirus or the Parapoxvirus vector according to the invention apply to the use of the invention in a corresponding manner.

It is to be understood that the previously mentioned features and those to be explained in the following cannot only be used in the combination indicated in each case, but also in other combinations or in isolated position without departing from the scope of the invention.

### EMBODIMENTS

The invention is now further explained by means of embodiments resulting in additional features, characteristics and advantages of the invention. The embodiments are of pure illustrative nature and do not limit the scope or range of the invention. The features mentioned in the specific embodiments are features of the invention and may be seen as general features which are not applicable in the specific embodiment but also in an isolated manner in the context of any embodiment of the invention. Reference is made to the enclosed figures where the following is shown.

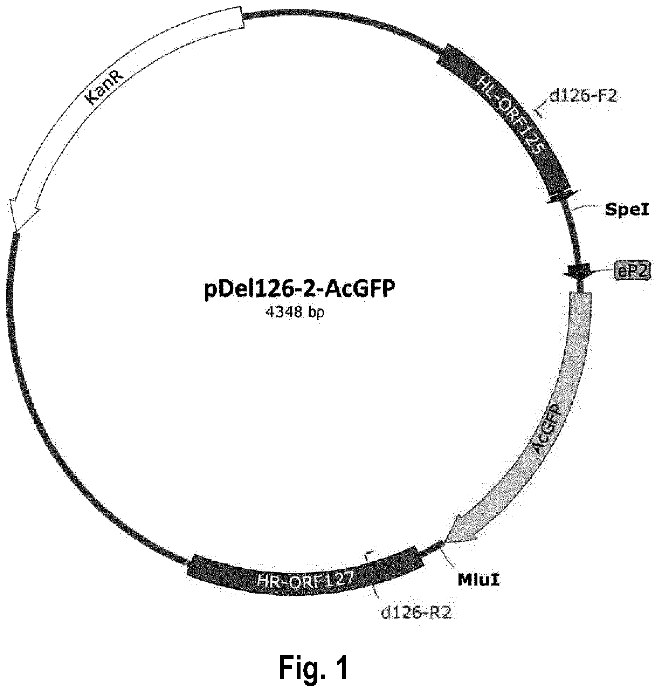

- Fig. 1:: Plasmid chart of pDel126-2-AcGFP.
- Fig. 2:: Graphical description of ORF126 deletion in the D1701-V genome. A) Shows the genomic map of ORFV strain D1701-V as recently published by Rziha et al. (2019; *I.c.*). B) Open reading frame (ORF) encoded by the right-hand part of the genome; the 31.805 nt comprising DNA sequence was determined by Rziha, H.-J., unpublished data. C) Enlargements of the genomic sites affected by each gene deletion. The corresponding sequences are given in SEQ ID NO: 6.

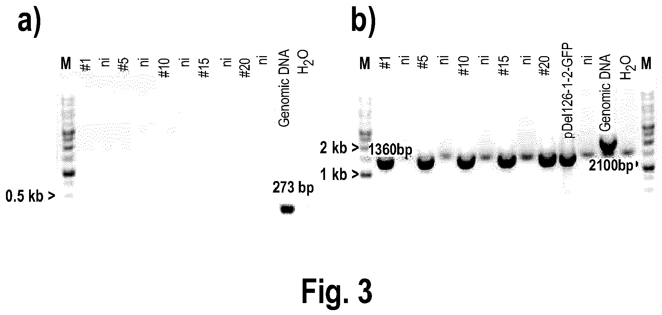

- Fig. 3:: GFP is stably integrated into Del126. 126 PCR after passage 1, 5, 10, 15 and 20 of VCh126GFP in Vero cells indicates a constant deletion of gene 126 (A), while d126 PCRs result in 1360 bp fragments specific for GFP inserted into the Del126 locus (B). 1% agarose gel; ni = DNA from not infected Vero cells; M = Ready-To-Use 1 kb Ladder, Nippon Genetics.

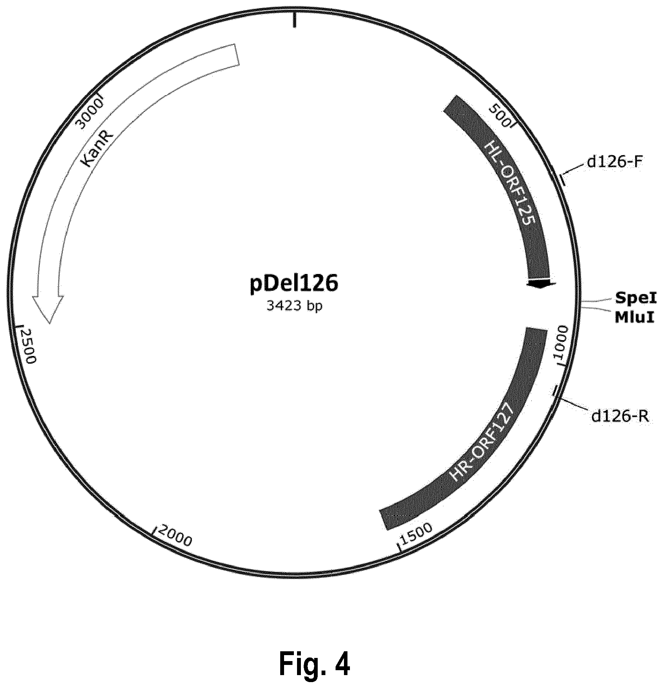

- Fig. 4:: Plasmid chart of pDel126.

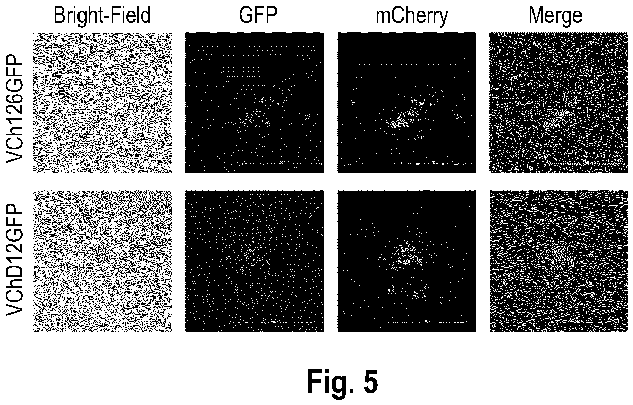

- Fig. 5:: New Del-site recombinant VCh126GFP induces the expression of GFP and mCherry in Vero cells. Fluorescence microscopy of single plaques was performed five days after infection with the new Del-site recombinant and the reference virus VChD12GFP. Pictures obtained from bright field microscopy, single GFP and mCherry channels as well as merged pictures are shown, while scale bars represent 500 µm.

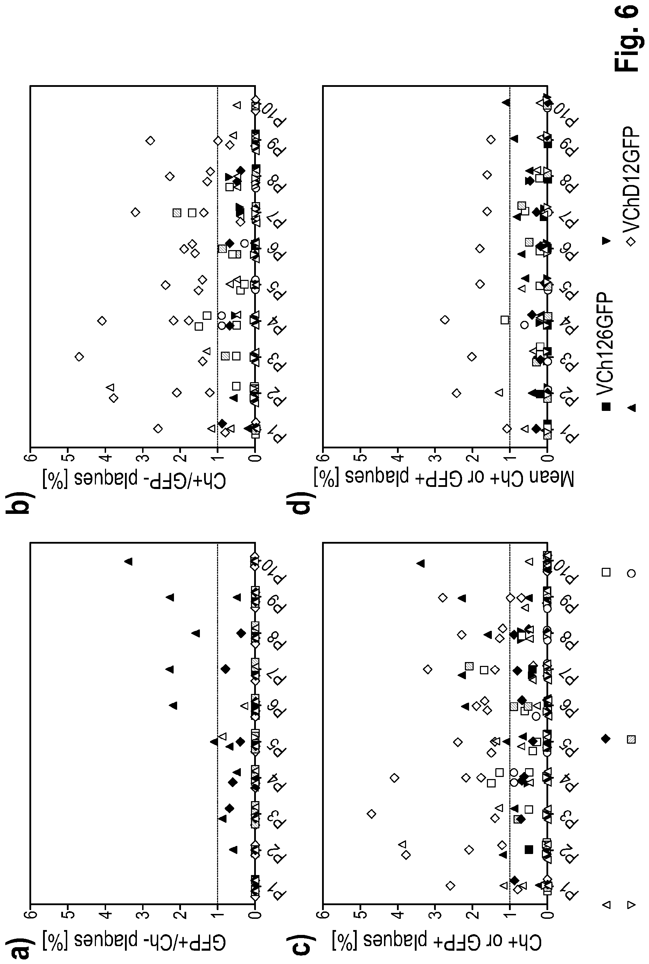

- Fig. 6:: Genetic stability of transgenes gfp and mcherry in Del-site recombinant VCh126GFP. During ten serial passages (P1-P10), the expression of fluorophores by infected Vero cells was determined by single plaque counting after 72 h post infection. Frequencies of single GFP (A) and single mCherry (B) expressing plaques, as well as the overall frequency of plaques showing single fluorescence (C) are shown for three viral clones per Del-site recombinant obtained from 96-well limiting dilutions. The mean overall frequency of single fluorescent plaques was calculated and plotted in (D).

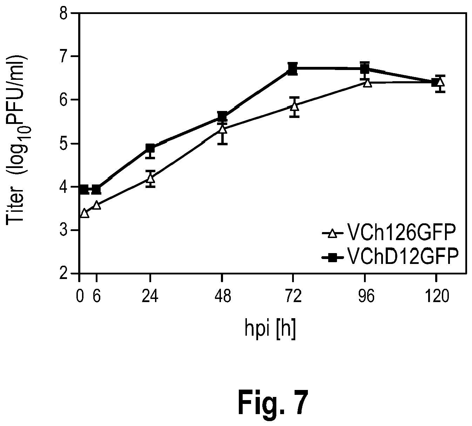

- Fig. 7:: Single step growth curves of Del-site recombinants. Infected cells (MOI 1) were washed and harvested 2 h after adsorption (0 hpi) and at the indicated hours post infection (hpi), while total cell lysates were titrated on Vero cells to determine the virus titer (PFU/ml). The virus growth curve of the recombinant VCh126GFP reaches comparable virus titers than the control VChD12GFP after 120 h.

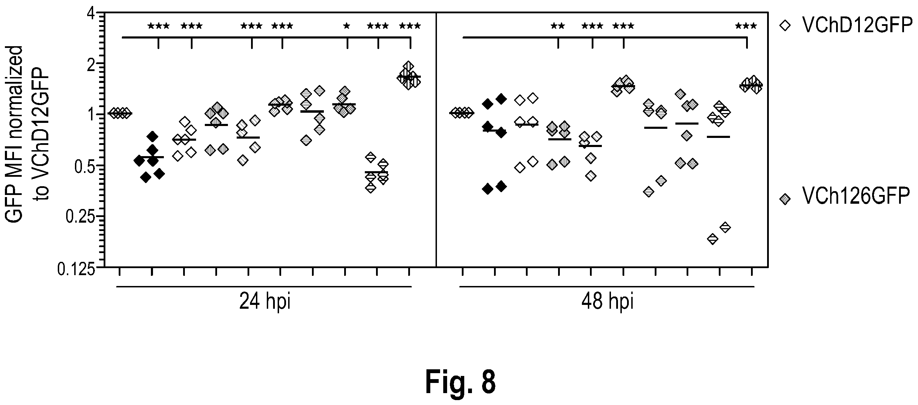

- Fig. 8:: Comparison of GFP and mCherry expression in Vero cells. Vero cells were infected with new Del-site recombinant VCh126GFP and the reference virus VChD12GFP, and harvested 24 hpi and 48 hpi. GFP geometric mean fluorescence intensities (MFI) were determined by FACS analysis and normalized to that of VChD12GFP. Shown are duplicates of three independent experiments. Statistical analysis was performed using an unpaired t-test with a confidence interval of 95%: ns = p ≥ 0.05, * = p < 0.05, ** = p < 0.01, *** = p < 0.001.

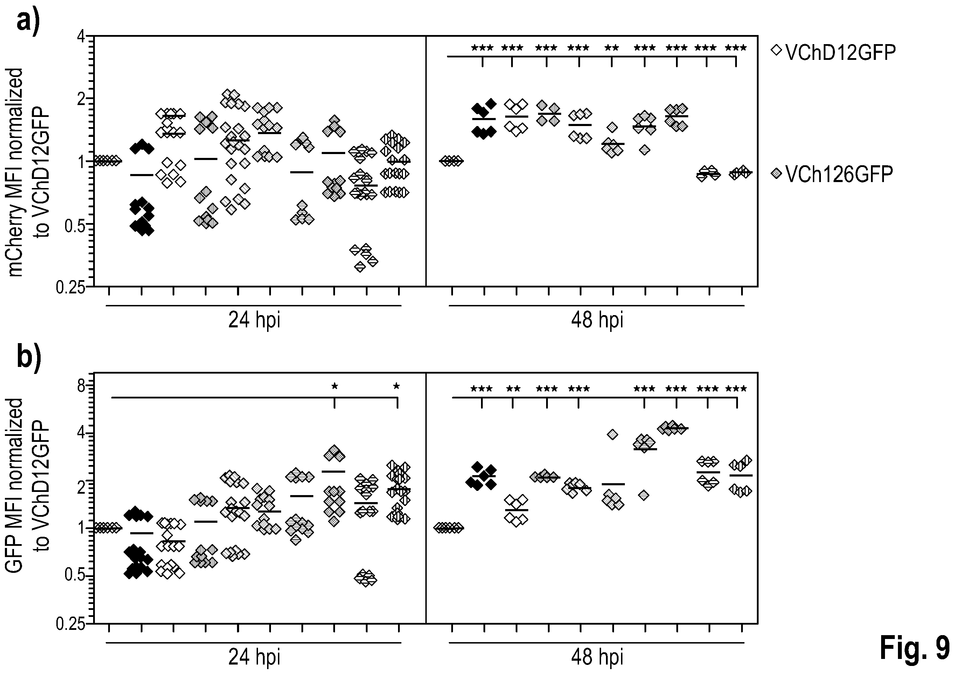

- Fig. 9:: Comparison of GFP and mCherry expression in monocytic THP-1 cells. THP-1 cells were infected with the new Del-site recombinant VCh126GFP and the reference virus VChD12GFP, and harvested 24 hpi and 48 hpi. mCherry (A) and GFP (B) geometric mean fluorescence intensities (MFI) were determined by FACS analysis and normalized to that of VChD12GFP. Mean values of each independent experiment were calculated to perform statistical analysis using an unpaired t-test with a confidence interval of 95%: ns = p ≥ 0.05, * = p < 0.05, ** = p < 0.01, *** = p < 0.001.

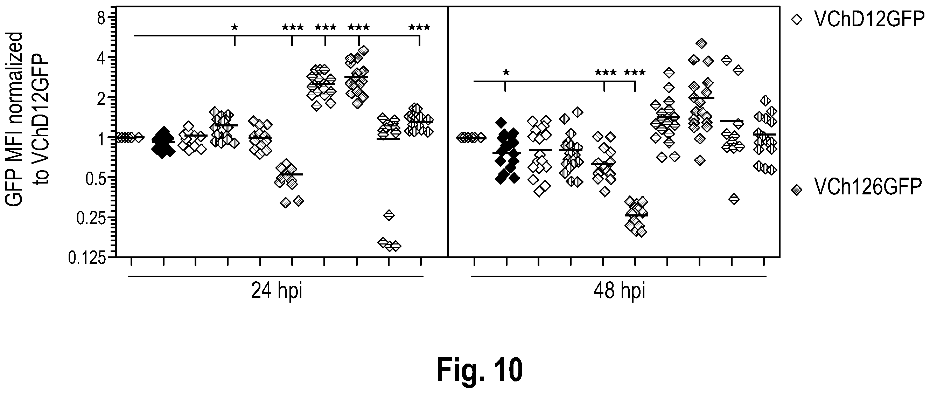

- Fig. 10:: Comparison of GFP and mCherry expression in moDCs. moDCs were infected with the new Del-site recombinant VCh126GFP and the reference virus VChD12GFP, and harvested 24 hpi and 48 hpi. GFP geometric mean fluorescence intensities (MFI) were determined by FACS analysis and normalized to that of VChD12GFP. Statistical analysis was performed using an unpaired t-test with a confidence interval of 95%: ns = p ≥ 0.05, * = p < 0.05, ** = p < 0.01, *** = p < 0.001.

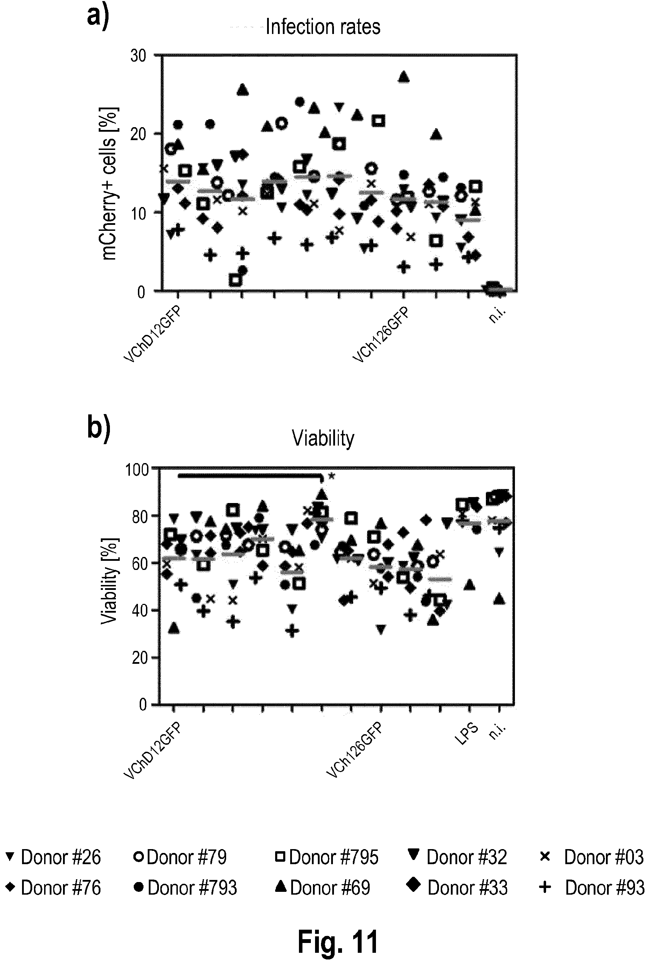

- Fig. 11:: Infection rates and viability of human moDCs. moDCs of ten different donors were infected with VCh126GFP and the reference recombinant VChD12GFP. Shown are the percentages of infected and viable cells determined by mCherry expression (A) and Zombie Aqua staining (B) for each of the five independently performed experiments and means, respectively. Statistical analysis was performed using a paired t-test with a confidence interval of 95%: ns = p ≥ 0.05, * = p < 0.05, ** = p < 0.01, *** = p < 0.001.

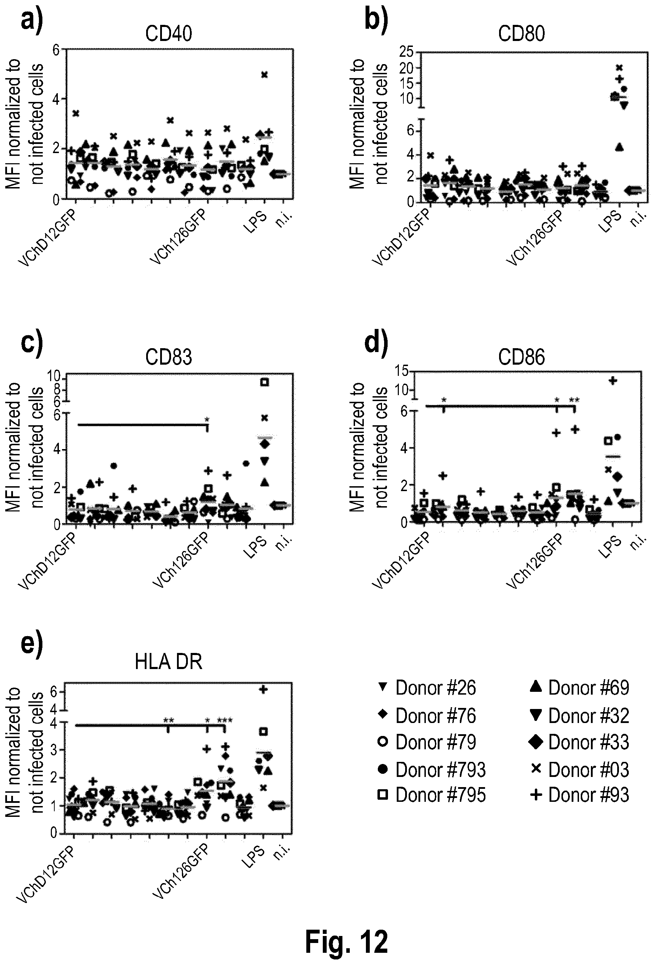

- Fig. 12:: Activation of human moDCs by the new Del-site recombinant VCh126GFP. moDCs of five different donors were infected with VCh126GFP and the reference recombinant VChD12GFP. The percentage of infected cells was determined by mCherry expression (A), while normalization to not infected cells allowed analyses of the relative activation of moDCs by CD40 (B), CD80 (C), CD83 (D), CD86 (E) and HLA-DR (F) expression via flow cytometry. Shown are the changes in expression strengths for each of the five independently performed experiments and means. Statistical analysis was performed using a paired t-test with a confidence interval of 95%: ns = p ≥ 0.05, * = p < 0.05, ** = p < 0.01, *** = p < 0.001.

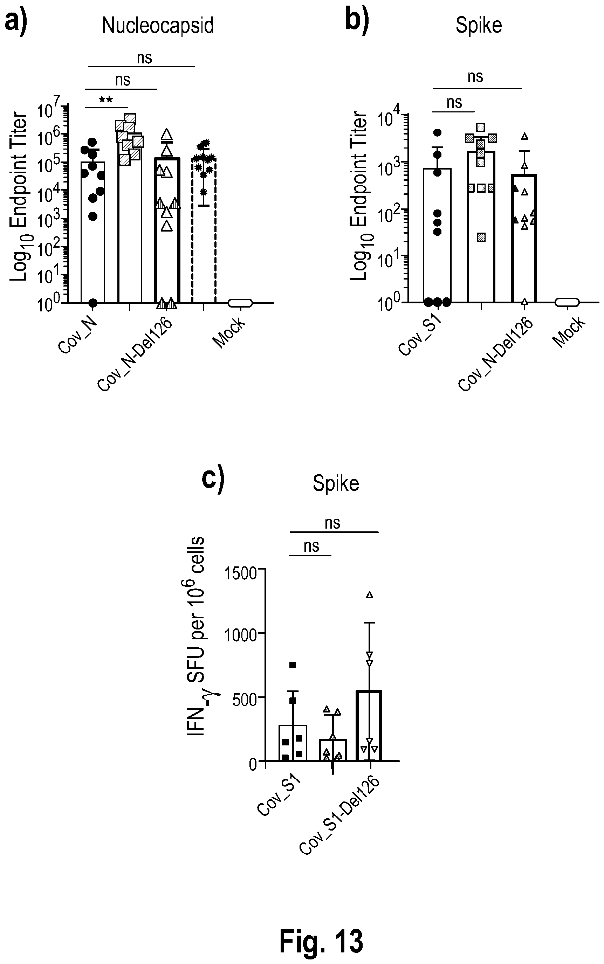

- Fig. 13:: Humoral and cellular immune responses stimulated by SARS-CoV-2 ORFV recombinants. CD1 mice were immunized twice in a two week interval with 107 PFU of control ORFV recombinants expressing N- or S1-SARS-CoV-2 proteins, or with the ORFV vectors containing an ORF126 deletion. A) - B) N- and S1 -specific binding total IgG induced by the ORFV vectors were evaluated after the second immunization by ELISA. C) S1 -specific cellular responses elicited by the ORFV vectors in splenocytes measured by IFN-γ ELISPOT assay. One-way ANOVA with Dunnett's multiple comparison test was used to evaluate differences between binding antibody endpoint titers or IFN-γ spot forming units (SFU) compared to the control ORFV group.

### 1. Preliminary remark

The present invention relates to the use of modified Parapoxviruses or Parapoxvirus vectors as a tool for the induction of an increased immune response in living beings and, in a further development, the expression of a trans- or foreign gene. To allow the insertion of multiple transgenes into a single Parapoxvirus vector, the present invention also relates to the suitability of the open reading frame (ORF) encoding the 'Ankyrin Repeat 1' (ANK-1), such as ORF126 in Parapoxvirus ovis (Orf virus, ORFV) stain D1701 and D1701-V, as insertion site for transgene expression. The novel deletion mutants were subjected to detailed characterization of the genetic stability of inserted AcGFP (GFP) reporter constructs, their growth behavior and capability to induce transgene expression in different target cells in vitro. Additionally, the mutants' immunogenicity was analyzed by its ability to activate peripheral blood mononuclear cells and antigen presenting cells, or to induce antigen-specific immune responses in vitro and in vivo. Taken together, the analyses performed demonstrate a high potential for the design of polyvalent, single vectored vaccines by integrating knockouts of ANK-1-encoding ORFs into the Parapoxvirus genome. Thus, the exchange of the open reading frame with the reporter gene GFP resulted not only in efficient replication of stable vectors expressing the desired transgene, but also attributed remarkable immunogenicity properties to the newly generated recombinants.

### 2. Introduction

The ORFV strain D1701-V was obtained from the bovine kidney cell line BK-KL3A adapted strain D1701-B and showed several genomic rearrangements after adaptation for growth in the African green monkey cell line Vero; Cottone, R., et al. (1998), Analysis of genomic rearrangement and subsequent gene deletion of the attenuated Orf virus strain D1701, Virus Research 56(1), p. 53-67; Rziha, H.J., et al. (2000), Generation of recombinant parapoxviruses: non-essential genes suitable for insertion and expression of foreign genes, Journal of Biotechnology 83(1), p. 137-145. These genomic rearrangements include the deletion of genes that are non-essential for the strain's replication and were shown suitable for transgene expression such as the deleted region D (D locus); Rziha, H.-J., et al. (2019; *I.c.*). Furthermore, the angiogenic factor VEGF-E was predicted a major virulence determinant responsible for the induction of bloody lesions in sheep Rziha, H.J., et al. (2000; *I.c*.) and thus, used as insertion site to generate ORFV recombinants triggering long-lasting immunity with a high protective efficacy against several viral diseases in different hosts. Besides these successfully used insertion sites, other genes potentially suited for transgene expression have been identified in the terminal ends of the D1701-V genome. As the central regions of poxviruses generally encode for essential genes conserved in position, spacing and orientation, these regions encode for factors influencing virulence, pathogenesis or host range and are considered dispensable for in vitro growth. While these factors are predicted to interfere with the optimal induction of cellular and humoral immune responses triggered by the host, a deletion of these immunomodulatory genes may further enhance the immunogenicity of the D1701-V vector. Thus, the present work focuses on the deletion of the gene encoding the 'NF-κB inhibitor' and its use as insertion site for transgene expression.

### Ankyrin Repeat 1 (ANK-1) - ORF126

'Ankyrin Repeat 1' (ANK-1) belongs to a group of protein motives consisting of two alpha helices separated by loops, first discovered in signaling proteins in yeast Cdc10 and Drosophila Notch. However, Ankyrin Repeats (ANK) are ubiquitous throughout the kingdoms of life, however, absent in most viruses except the poxvirus family, specifically the Chordopoxviruses; see Herbert, M.H. et al. (2015), Poxviral ankyrin proteins. Viruses 7(2): p. 709-38. The motif consists of tandem repeated consensus domains that are crucial to facilitate protein-protein interactions and is found in four proteins encoded by ORFV D1701-V (ORF123, -126 (ANK-1), -128 (ANK-2) and -129 (ANK-3)); see Rziha, H.-J., et al. (2019), Genomic Characterization of Orf Virus Strain D1701-V (Parapoxvirus) and Development of Novel Sites for Multiple Transgene Expression. Viruses 11(2): p. 127; and Rziha, H.J., et al. (2003), Relatedness and heterogeneity at the near-terminal end of the genome of a parapoxvirus bovis 1 strain (B177) compared with parapoxvirus ovis (Orf virus). J. Gen. Virol. 84(Pt 5): p. 1111-6. While plenty of cellular ANK proteins are described to be involved in cell-cell signaling, cytoskeleton integrity, regulation of transcription and cell cycle, inflammatory response, or protein transport (Mosavi, L.K., et al. (2004), The ankyrin repeat as molecular architecture for protein recognition. Protein Sci. 13(6): p. 1435-48), only little is known about the function of poxviral ANK proteins. It has been demonstrated that these four proteins harbor F-box-like motifs that may recruit specific substrates to SCF1 ubiquitin ligases via a substrate-binding domain and thereby exploit the cell's ubiquitin-proteasome machinery; see Sonnberg, S., et al. (2008), Poxvirus ankyrin repeat proteins are a unique class of F-box proteins that associate with cellular SCF1 ubiquitin ligase complexes. Proc Natl Acad Sci U S A 105(31): p. 10955-60. One of these proteins, the ORF126 encoded ANK-1 protein, was shown to co-localize with the mitochondria via the essential ankyrin repeats 8 and 9. Nevertheless, the physiological consequences have not been demonstrated yet; see Lacek, K., et al. (2014), Orf virus (ORFV) ANK-1 protein mitochondrial localization is mediated by ankyrin repeat motifs. Virus Genes 49(1): p. 68-79. Furthermore, a recent study reported an influence of ORFV encoded ANK proteins on the Hypoxia-inducible factor (HIF) pathway; see Chen, D.Y., et al. (2017), Ankyrin Repeat Proteins of Orf Virus Influence the Cellular Hypoxia Response Pathway. J. Virol. 91(1). While this pathway plays a crucial role in the regulation of cellular responses to hypoxia, downstream targets like angiogenesis- and anti-apoptotic programs favor viral pathogenesis; see Cuninghame, S., R. et al. (2014), Hypoxia-inducible factor 1 and its role in viral carcinogenesis. Virology 456-457: p. 370-83. In their study, Chen et al. could show that the factor inhibiting HIF (FIH) was sequestered by ANK proteins upon ORFV infection resulting in enhanced HIF activity; see Chen et al. (2017; *I.c.*).

### 3. Material and methods

### Generation and characterization of new Del-site recombinants

In order to generate the ORFV recombinants used in this work, transgenes synthesized and obtained from Invitrogen were cloned into transfer plasmids. Stable integration of transgenes into the ORFV genome was accomplished by homologous recombination between the transfer plasmid and the genomic DNA of the parental virus following transfection by nucleofection and subsequent infection of Vero cells.

### Generation of transfer plasmids

DNA inserts as well as plasmid vectors were digested using the same restriction enzymes, fragments of interest were purified and ligated. Subsequently, bacteria were transformed with ligated plasmids and colonies selected to produce the transfer plasmids. For validation, control digests using restriction enzymes was followed by agarose gel electrophoreses, while DNA sequencing using insert specific primers confirmed correct insertion of the transgenes into the plasmid vector. The following table summarizes the transfer plasmid generated, while the plasmid chart and corresponding sequence (SEQ ID NO: 7) are shown in Figure 1.

| **Transfer Plasmid** | **Insert Vector** | **Parental Vector** | **Restriction digest** |
|---|---|---|---|
| **pDel126-2-AcGFP** | pD12-AcGFP | pDel126 | MIul / Spel (937+3411 bp) |

### Transfection of ORFV infected Vero Cells

The generation of recombinant ORFVs followed two steps, in which Vero cells were transfected with transfer plasmids prior to infection with ORFV. Transfection was performed by an electroporation-based transfection method called nucleofection. Due to homologous sequences on the transfer plasmid and the ORFV genome, the inserts could be integrated into the ORFV genome via homologous recombination. For this purpose, Vero cells were detached using trypsin, re-suspended in 5 ml NF stop solution and counted. For each transfection batch, 2.5 x 10⁶ cells were transferred into a 1.5 ml Eppendorf cup and centrifuged at 63 rcf for 10 min. The cell pellet was re-suspended in 100 µl transfection solution (transfection supplement and CLB transfection buffer of the Amaxa transfection kit mixed in a ratio of 1:4.5), supplemented with 2 µg plasmid DNA and transferred into a transfection cuvette. For transfection, the CLB Transfection Device was used. Subsequently, nucleofected cells were re-suspended in NF stop solution and transferred into a T25 cell culture flask containing 6 ml pre-warmed Vero cell medium using a Pasteur pipette. Cells were immediately infected with parental virus (MOI 1) for 4 hours at 37 °C and 5% CO₂, washed using 6 ml pre-warmed PBS and incubated in fresh Vero cell medium for 72 h at 37 °C and 5% CO₂. When virus plaques or a cytopathic effect (CPE) could be observed, cells were frozen and thawed three times at -80 °C and 37°C in a water bath, respectively, to disrupt the cells and release viral progeny.

### Selection of ORFV recombinants

Homologous recombination is an event occurring in a ratio of approximately 1:10000 between the insert of the transfer plasmid and the target region in the ORFV genome. To select for this rare event and to separate the recombinant ORFVs from parental viruses, different selection methods can be used.

### FACS based selection of ORFV recombinants

Selection by fluorescence activated cell sorting (FACS) is based on the loss or gain of a fluorescent label such as GFP or mCherry. For this purpose, 3 x 10⁵ Vero cells were seeded in a 6-well plate containing 3 ml Vero cell medium. Transfection lysate was used for infection in serial dilutions and took place for 20-24 h at 37 °C and 5% CO₂. To ensure an optimal selection process, cells with a low infection rate of approximately 1-5% were harvested and centrifuged for 5 min at 400 rcf. Subsequently, cells were washed thrice with 1 ml PBE and eventually re-suspended in 500 µl PBE. Single cell FACS sorting was performed into a 96-well plate containing 10⁴ Vero cells per well in 150 µl Vero cell medium using the BD FACSjazz (Biosciences). After 72 h of incubation at 37 °C and 5% CO₂, wells showing single virus plaques of recombinant ORFV could be picked for further propagations and analyses.

### Selection of ORFV recombinants by limiting dilutions

Further selection and purification of recombinant viruses after FACS based sorting or MACS selection was carried out by limiting dilutions. For this purpose, 2 x 10⁶ Vero cells in 25 ml of Vero cell medium were split into a 12-well pipetting reservoir, in which the first and the rest of wells contained either 3 ml or 2 ml of the cell solution, respectively. The first well was supplemented with either 3 x 10⁵ cells of the MACS selection or 50-100 µl of virus lysate and diluted 1:3 each from the first to the last well. 150 µl of each dilution were transferred into the corresponding wells of a 96-well plate and incubated at 37°C and 5% CO₂ for 72 h. After 72 h, wells containing single virus plaques could be selected for further processing by (fluorescence-) microscopy.

### Determination of genetic stability by serial passages

To investigate the genetic stability of fluorophore coding sites, ten serial passages of respective ORFV recombinants were performed. For this, 5 x 10⁵ Vero cells were seeded in 6-well plates containing 3 ml Vero cell medium. At first, virus lysates showing one single plaque in a limiting dilution were used for infection in serial dilutions and took place for 2 h at 37 °C and 5% CO₂. Cells were washed twice with PBS and were subsequently incubated in 3 ml Vero cell medium for 72 h at 37 °C and 5% CO₂. Fluorescent plaque counts were determined for wells showing 100-200 plaques using the Eclipse Ti2 microscope (Nikon). Next, cells were frozen at -80 °C, thawed at RT and 50 µl of virus lysates were used to infect freshly seeded Vero cells as described above.

### Isolation of PBMCs from donated blood

Peripheral blood mononuclear cells (PBMCs) could be isolated from blood donations obtained from the blood donation center Tübingen. First, the blood was diluted with PBS to a total volume of 100 ml. Next, 4 x 15 ml Ficoll in 50 ml tubes were overlaid with 25 ml of diluted blood each and centrifuged at 700 x g and RT for 20 min. Leukocytes, which could be identified by the formation of a white layer after density gradient centrifugation, were transferred into two 50 ml tubes and each were supplemented with PBS up to a volume of 50 ml. After centrifugation at 400 x g and RT for 10 min, the cell pellets were re-suspended in 50 ml PBS and centrifuged at 300 x g and RT for 10 min. PBMCs were merged and supplemented with PBS up to a volume of 50 ml, and the cell number was determined.

### Isolation of Monocytes from PBMCs

The principle of isolating monocytes from PBMCs relies on the expression of CD14 by monocytes. Hence, PBMCs were centrifuged at 300 x g and RT for 10 min and the pellet was re-suspended in 4 ml PBE and 100 µl of α-CD14 MicroBeads. The suspension was incubated at 4°C for 15 min and loaded onto a LS column equilibrated with PBE. The column was washed three times with 3 ml PBE before the CD14+ monocytes could be eluted from the column with 5 ml PBE and counted.

### Differentiation of Monocytes to moDCs

The differentiation of purified monocytes to dendritic cells (moDCs) was carried out for five days by the stimulation with 86 ng/ml GM-CSF and 10 ng/ml IL-4 at 37 °C and 5% CO₂.

### Flow cytometry

The viability and infection rates, as well as the expression of surface and intracellular molecules was analyzed using the BD LSRFortessa flow cytometry system (BD Biosciences). The preparation of samples and staining of cells with fluorescence-labeled antibodies was carried out in 96-well U-bottom plates, while centrifugation occurred at 4°C and 400 x g for 5 min. For the determination of infection rates, cells were washed twice in 200 µl PFEA, and were re-suspended in 50 µl PFEA or optionally fixed for FACS analyses.

### Inhibition of Fc receptors

To prevent non-specific antibody binding, cells expressing Fc receptors were treated with Fc-block according to manufacturers' instructions prior to staining procedures.

### Staining with multimeres

Multimeres are frequently used to identify and quantify antigen-specific T cells. Tetramers consist of four recombinant MHC molecules conjugated to a fluorescently labeled streptavidin complex. In contrast, dextramers consist of a dextran backbone that carries several fluorophores and MHC molecules. Multimeres can be loaded with peptides of interest to form peptide-MHC complexes recognized by specific T cells, and thus, be detected by flow cytometry. For this, cells were washed twice with 200 µl of PBS and re-suspended in 50 µl of tetramer solution or PBS, respectively. Prior to staining with 50 µl of tetramer solution, PE-conjugated HLA tetramers were mixed 1:50 with tetramer buffer and centrifuged at 13.000 x g for 10 min. Staining with dextramers followed the same procedure by mixing dextramers in a ratio of 1:10 with PBS. The incubation was carried out at RT for 30 min in the dark.

### Determination of cell viability

Cell viability was determined by Zombie Aqua staining. Zombie Aqua is an amine-reactive fluorescent dye that cannot penetrate live cells but enters cells with compromised membranes. Therefore, it can be used to differentiate between live and dead mammalian cells. For Zombie Aqua staining, cells were washed twice with 200 µl PBS and re-suspended in 50 µl of Zombie Aqua diluted 1:400 in PBS for 30 min at 4°C in the dark.

### Extracellular antibody staining

For the analysis of surface molecule expression, cells were washed twice with 200 µl PFEA, re-suspended in 50 µl of freshly prepared antibody mix in PFEA and incubated for 30 min at 4 °C in the dark.

### Intracellular cytokine staining

For the detection of intracellular cytokines, the cells were treated with 10 µg/ml Brefeldin A to prevent secretion of proteins and stimulated with respective synthetic peptides for 12-14 h. Prior to intracellular cytokine staining, cells were washed twice with 200 µl PFEA and permeabilized by incubation with 50 µl Cytofix/Cytoperm for 30 min at 4 °C in the dark. Next, cells were re-suspended and washed twice with 200 µl Permwash and subsequently stained with 50 µl antibody mix in Permwash for 30 min at 4 °C in the dark.

### Fixation of cells

For storage exceeding 4 h, cells were fixed. For this, cells were washed twice with 200 µl PFEA, re-suspended in 50 µl PFEA + 1% formaldehyde and stored at 4 °C in the dark. Analysis of samples was carried out within 10 days.

### Mouse immunization

Outbred CD1 mice (N=10) of at least 6 weeks of age were obtained from Charles River (Charles River Laboratories, Germany) and were housed in the biosafety level 1 animal facility at the University of Tübingen, Germany. All animals were handled in strict accordance with good animal practice and complied with the guidelines of the local animal experimentation and ethics committee - experiments were conducted under Project License (Nr. IM 1/20G). Mice were immunized intramuscularly (i.m.) in the anterior tibialis with 10⁷ plaque forming units (PFU) of ORFV recombinants twice with a two weeks interval. Blood samples for humoral immune analysis were collected by retro-orbital bleeding two weeks post booster immunization. Splenocytes for cellular immune analysis were collected two weeks post booster immunization and were isolated by standard procedure after animals were humanely euthanized.

### IFN-γ ELISPOT assay

The ELISPOT assay was performed using the mouse IFN-γ ELISpot PLUS kit (ALP) (Catalog #: 3321-4APW-10, Mabtech, Schweden) according manufacturer's instructions. 2×105 splenocytes per well were stimulated with the overlapping pools of S1 peptides with the final concentration of 2µg/ml (Catalog #: 130-127-041, Miltenyi, Germany) for 21 h. Developed spots were automatically counted using an ImmunoSpot S5 analyzer (Cellular Technology Limited, USA) and ImmunoSpot software.

### 4. Results

### Generation of new Del-site recombinants

After the successful generation of transfer plasmids, the new ORFV recombinant was generated as described in the methods in detail. Thus, the plasmid pDel126-2-AcGFP was transferred into Vero cells by nucleofection, which were subsequently infected with the parental virus V12-Cherry. Due to homologous sequences in the ORFV genome and the insert flanking regions of the transfer plasmid, homologous recombination led to stable exchange of ORF126 by GFP in the ORFV D1701-V genome (Figure 2). The exact position of ORF126 in the right-hand part of the 31.805 nt comprising DNA sequence of the D1701-V (Rziha, H.-J., unpublished, Figure 2B) is shown in the following table:

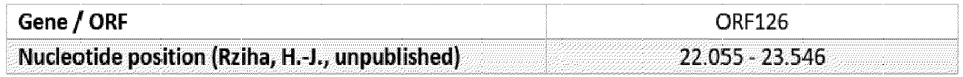

Next, new ORFV recombinants could be isolated from infected GFP and mCherry expressing Vero cells selected by FACS sorting and limiting dilutions. DNA isolation followed by insert and locus specific PCR typing allowed monitoring of the genetic homogeneity of the purified ORFV recombinants as shown in Fig. 3. Here, 126 and d126 PCR proved both, the deletion of the ORF126 and exchange with the GFP encoding sequence. Subsequently, the new ORFV recombinants were propagated in large scale as described above.

Recombinant ORFV deletion mutants could also be generated, which only led to the deletion of ORF126 without the insertion of GFP and thus, a loss of functional ORF126 gene product leading to enhanced immunogenicity compared to the parental virus. The corresponding transfer plasmid and sequence can be found in Figure 4 and SEQ ID NO: 8.

### GFP is stably inserted into the new Del-Sites

To investigate the stability of inserted GFP into the new Del-sites, Vero cells were infected with the new Del-site recombinant VCh126GFP and the reference virus VChD12GFP and 20 serial passages were performed. Both, GFP and mCherry fluorescence could be observed by fluorescence microscopy in single plaques resulting from Vero cell infection with the new Del-site recombinant throughout this experiment as shown in Figure 5.

Additionally, viral DNA was isolated from infected Vero cells after Passage 1, 5, 10, 15 and 20, and deleted gene-specific as well as locus-specific PCRs were performed to examine the Del-locus' integrity. As shown, no ORF126 specific 273 bp fragments could be detected in 126 PCRs during 20 passages (Figure 3A), while the deletion site showed 1360 bp fragments specific for GFP inserted into the Del126 locus using the d126 PCR (Figure 3B).

Finally, the stability of transgenes inserted into new Del-sites was examined during ten serial passages of three biological replicates (virus clones) of the ORFV D1701-V recombinants. After 72 h of infection, wells containing 100-200 plaques were used to determine the amount of single plaques expressing both, GFP and mCherry fluorophores, or either GFP or mCherry by fluorescence microscopy. The percentages of plaques expressing only one fluorophore during ten serial passages are shown in Figure 6.

The results demonstrate frequencies of less than 1% single GFP and mCherry expressing plaques using VCh126GFP. Taken together, the results presented suggest that GFP is stably expressed in Vero cells infected with the new Del-site recombinant and can be detected throughout 20 passages in the predicted genomic locus. Furthermore, the genetic stability of both, GFP and mCherry in the new Del-site or the vegf-locus, respectively, was validated in ten serial passages studying the fluorescence of infected Vero cells. Since at least 99 % of all counted lytic plaques infected with each new Del-site recombinant expressed GFP and mCherry simultaneously after ten passages, these results indicate genetic stability of the examined genomic loci.

### Characterization of new Del-site recombinants' growth behavior

To investigate, whether the deletion introduced into the new Del-site recombinant alters the *in vitro* growth characteristics in the ORFV permissive Vero cells compared to the reference virus VChD12GFP, single-step growth curve experiments were performed. For this, Vero cells were infected with the new Del-site recombinant VCh126GFP leading to infection rates of approximately 20-25% after 24 h. Infected cells were washed and harvested 2 h after adsorption (designated 0h) or 6 h, 24 h, 48 h, 72 h, 96 h and 120 h post infection. Virus lysates were titrated on Vero cells to determine the number of infectious particles measured in plaque forming units per ml (PFU/ml). The experiment was performed three times for each recombinant and the results are shown in Figure 7A-I.

The growth characteristics of Del-site recombinant VCh126GFP resembles the ones obtained for the reference virus VChD12GFP reaching comparable final titers of approximately 10⁷ PFU/ml after 120 hpi. Notably, the growth curve of VCh126GFP indicates lower virus input as used for the growth curve of VChD12GFP and showed only slightly diminished final virus titers compared to the control throughout the experiment (Figure 7). Nevertheless, these results suggest VCh126GFP to be replication efficient as it was able to produce infectious progeny in the permissive cell line Vero.

### Analyses on expression strengths using new Del-Sites

To investigate the potential of transgene expression by the new Del-site recombinant, the reporter gene GFP integrated into the ORF126 and mCherry inserted into the vegf-locus were used to compare their expression strength after ORFV infection of Vero cells, the monocytic cell line THP-1 and of human primary monocyte derived dendritic cells (moDCs). For this, cells were seeded and infected VCh126GFP and the reference virus VChD12GFP for 24 h or 48 h. Subsequently, FACS analysis was performed and geometric mean fluorescent intensities (MFI) of GFP and mCherry expressed in the cells were determined. For the analysis of moDCs, experiments were performed with cells from several donors to estimate the differences between individuals. MFIs identified from new Del-site recombinants' infections were normalized to the mean of VChD12GFP derived MFIs of the same experiment. Results obtained from Vero cells, THP-1 cells and moDCs are shown in Figure 8, Figure 9 and Figure 10, respectively.

For Vero cells, three independent experiments were performed in duplicates. As shown in Figure 8, GFP levels induced by VCh126GFP were significantly enhanced after 24 h compared to reference virus VChD12GFP.

The expression strength of GFP and mCherry in monocytic THP-1 cells was analyzed in four independent experiments including three to six replicates 24 h after infection, and in two independent experiments including triplicates 48 h after infection, respectively. Here, significantly increased mCherry could be detected for VCH126GFP infected THP1 cells compared to those infected with the reference virus after 48 h (Figure 9A). Additionally, the GFP expression was significantly accelerated for VCh126GFP compared to VChD12GFP after 24 h and 48 as demonstrated in Figure 9B. Considering these elevated transgene levels in comparison to VChD12GFP, the deletion of ORF126 seems to facilitate the expression of transgenes integrated into the vegf-locus, whereas transgenes inserted into ORF126 are expressed stronger than those encoded by the D-locus in THP-1 cells.

To study the expression of transgenes compared to the reference virus VChD12GFP in primary cells, moDCs were generated and infected. For each of the three donors, the GFP and mCherry MFIs of six technical replicates were evaluated by FACS 24 h and 48 h after infection. As demonstrated in Figure 10, GFP expression in moDCs infected with VCh126GFP was significantly increased after 24 h and elevated after 48 h and thus, to enhance expression levels compared to those resulting from the D-locus in moDCs.

In conclusion, the analyses on expression strengths show that the deletion of selected ORFs encoded by ORFV D1701-V and their replacement with gfp causes alterations in the expression of transgenes inserted into the vegf-locus. Especially the deletion of ORF126, which encodes ANK-1 seemed to affect the expression kinetics of inserted transgenes. While the expression of the fluorescent reporters GFP and mCherry induced by most of the new Del-site recombinants appeared to be decreased, comparable or higher expression levels could be achieved in THP-1 and moDCs upon VCh126GFP infection.

### Activation of Dendritic Cells by Infection with new Del-site Recombinants

Previously, Müller et al. (2019, in preparation) could show that the uptake of D1701-V ORFV recombinants encoding different transgenes can alter the activation of antigen presenting cells (APCs) such as dendritic cells or monocytes within PBMCs. To elucidate the impact of an ORF126 deletion introduced into the D1701-V genome, moDCs were infected with VCh126GFP and the reference virus VChD12GFP, while not infected cells served as negative control. After 24 h, cells were harvested to determine the expression of the activation markers CD40, CD80, CD83, CD86 and HLADR of mCherry expressing cells that showed most comparable infection rates within one donor by FACS analysis. LPS treated and not infected cells served as controls, whereas their activation marker expression was measured in mCherry negative cells.

In total, the activation marker expression was determined in infected moDCs derived from ten healthy donors. Infection rates of moDCs from different donors ranged from 5% - 20%, which in mean leveled between 10% to 15% (Figure 11A). The mean viability of infected moDCs did not correlate with infection rates and leveled at approximately 60%. To compare these data, the expression of activation markers was normalized to the geometric mean fluorescence intensity obtained from not infected cells. The results shown in Figure 12A and B indicate that the relative expression of the activation markers CD40 and CD80 did not differ significantly upon infection with the reference virus VChD12GFP or VCh126GFP. The expression of CD83, CD86 and HLADR, however, was significantly increased after infection of moDCs with VCh126GFP as illustrated in Figure 12D, E and F. In summary, the deletion of ORF126 in ORFV D1701-V recombinants positively impacts the surface expression of analyzed activation markers.

### In vivo immunogenicity of SARS-CoV-2 ORFV recombinants

The immunogenicity of SARS-CoV-2 ORFV recombinants encoding for the Spike- (S1) and Nucleoprotein (N) was compared in outbred CD1 mice following two intramuscularly (i.m.) immunizations. High-titer N- and S1- antigen-specific binding antibodies were detected in all vaccine groups after the booster immunization (Figure 13). While ORFV recombinants with ORF126 knockouts induced SARS-CoV-2 N- and S1-specific binding antibody titers comparable to the control ORFV (Figure 13A and B), mice receiving prime-boost immunization with an ORF126 knockout mutant exhibited highest numbers of IFN-γ producing T-cells compared to two other groups (Figure 13C).

### 5. Summary

Taken together, the analyses on the new Del-site recombinants performed demonstrate a high potential for the design of polyvalent, single vectored vaccines by integrating an ORF126 knockout into the ORFV D1701-V genome. The deletion of ORF126 and simultaneous integration of the reporter construct GFP into the respective Del-site resulted not only in efficient replication of stable vectors expressing the desired transgenes, but also attributed remarkable immunogenicity properties to the newly generated recombinants. Accordingly, the deletion mutant lacking ORF126 encoding for ANK-1 was shown to stimulate an enhanced expression of moDC activation markers in vitro and elicited similar humoral and superior cellular immune responses compared to the reference mutant in vivo. Moreover, expression kinetics on GFP inserted into the Del-site 126 lead to the strongest reporter levels indicating its suitability for strong transgene expression. Thus, the findings of the present study may pave the way for the generation of ORFV D1701-V vectored vaccines with favorable properties, in which recombinants harboring an ORF126 deletion simultaneously expressing several antigens and immuno-modulatory elements from the vegf-locus and Del-site 126 lead to the induction of strong, long-lasting and efficient cellular as well as humoral immune responses.

### 6. Sequences

| | |
|---|---|
| SEQ ID NO: 1: | Nucleotide sequence of eP1 promoter |
| SEQ ID NO: 2: | Nucleotide sequence of eP2 promoter |
| SEQ ID NO: 3: | Nucleotide sequence of "optimized early" promoter |
| SEQ ID NO: 4: | Nucleotide sequence of 7.5 kDa promoter |
| SEQ ID NO: 5: | Nucleotide sequence of VEGF promoter |
| SEQ ID NO: 6: | Nucleotide sequence of D1701-V ORF125 - 126 - 127 (nt 1 - 2380) |
| | ORF125: nt 1 - 519 |
| | ORF126: nt 629 - 2119 |
| | ORF120: nt 2204 - 2758 |
| SEQ ID NO: 7: | Nucleotide sequence pDel 126-4-2-AcGFP (nt 1 - 2057) |
| | HL (homolog left arm) ORF125: nt 1 - 447 |
| | Early promoter eP2: nt 632 - 668 |
| | AcGFP: nt 700 - 1416 |
| | HR (homolog right arm) ORF127: nt 1488 - 2057 |
| SEQ ID NO: 8: | Nucleotide sequence pDel-126 (nt 1 - 1132) |
| | HL (homolog left arm) ORF125: nt 1 - 447 |
| | HR (homolog right arm) ORF127: nt 563 - 1132. |

## Claims

1. A modified *Parapoxvirus* comprising at least one functional mutation in the viral open reading frame (ORF) encoding an 'Ankyrin Repeat 1' (ANK-1), wherein said virus comprises increased immunogenicity in comparison with the same vector without said functional mutation.

2. The modified *Parapoxvirus* of claim 1, wherein said functional mutation results in a reduction of the activity of ANK-1 over non-mutated ANK-1.

3. The modified *Parapoxvirus* of claim 1 or 2, which is a *Parapoxvirus* vector.

4. The modified *Parapoxvirus* or *Parapoxvirus* vector of any of the preceding claims, wherein the *Parapoxvirus* is a *Parapoxvirus ovis* (*Orf* virus, ORFV) or the *Parapoxvirus* vector is an ORFV vector.

5. The modified *Parapoxvirus* or *Parapoxvirus* vector of claim 4, wherein said ORFV is of the strain D1701, preferably of the strain D1701-V.

6. The modified *Parapoxvirus* or *Parapoxvirus* vector of any of the preceding claims, wherein said ANK-1 is encoded by viral open reading frame 126 (ORF126), preferably said ORF is located at the nucleotide positions nt 22.055 ± 100 to nt 23.546 ± 100.

7. The modified *Parapoxvirus* or *Parapoxvirus* vector of any of the preceding claims, further comprising:
(1) at least one nucleotide sequence encoding a transgene, and
(2) at least one promoter controlling the expression of the transgene-encoding nucleotide sequence.

8. The modified *Parapoxvirus* or *Parapoxvirus* of claim 6, wherein said transgene-encoding nucleotide sequence is inserted into said ANK-1-encoding viral ORF, or/and wherein said ANK-1-encoding viral ORF is replaced by said transgene-encoding nucleotide sequence.

9. The *Parapoxvirus* vector of any of claims 6-8, comprising more than one transgene-encoding nucleotide sequence, preferably the number of transgene-encoding nucleotide sequences is selected from the group consisting of: 2, 3, 4 or more.

10. The modified *Parapoxvirus* or *Parapoxvirus* vector of any of claims 6-9, wherein the promoter is an early ORFV promoter.

11. The modified *Parapoxvirus* or *Parapoxvirus* vector of claim 10, wherein the early ORFV promoter comprises a nucleotide sequence which is selected from the group consisting of: SEQ ID NO: 1 (eP1), SEQ ID NO: 2 (eP2), SEQ ID NO: 3 ("optimized early"), SEQ ID NO: 4 (7.5 kDa promoter), and SEQ ID NO: 5 (VEGF).

12. The modified *Parapoxvirus* or *Parapoxvirus* vector of any of the preceding claims, wherein the transgene is selected from the group of the following antigens:
- viral antigen, preferably
severe acute respiratory syndrome coronavirus 2 (SARS-CoV-2) antigen, including spike (S), envelope (E), and nucleocapsid (N) proteins;
rabies virus antigen, including glycoprotein (RabG);
influenza A antigen, including nucleoprotein (NP), hemagglutinin (HA), neuraminidase (NA);
- tumor antigen, preferably viral tumor antigen, including HPV selective viral tumor antigen;
- tumor associated antigen, including viral tumor associated antigen, including HPV selective viral tumor-associated antigen;
- parasitic antigen, preferably plasmodium antigen;
- cytokine;
- protein originated or derived from a mammal, preferably from a mammal recipient.

13. A biological cell containing the modified *Parapoxvirus* or *Parapoxvirus* vector of any of the preceding claims, preferably a mammalian cell, further preferably a Vero cell, a HEK 293 cell or an antigen-presenting cell.

14. A pharmaceutical composition, preferably a vaccine, containing the modified *Parapoxvirus* or *Parapoxvirus* vector of any of claims 1-12 or/and the cell of claim 13, and a pharmaceutically acceptable carrier.

15. Use of a modified *Parapoxvirus* or *Parapoxvirus* vector comprising at least one functional mutation in the viral open reading frame (ORF) encoding the ANK-1 for the induction of an immune response in a living being, preferably a mammal or a human being.
